Europäisches Patentamt

European Patent Office

Office européen des brevets

(11) Publication number: **0 430 356 A2**

## EUROPEAN PATENT APPLICATION

(21) Application number: 90203087.3

(51) Int. Cl.⁵: **B01L 3/14**

(22) Date of filing: 22.11.90

(30) Priority: 29.11.89 US 442826
16.05.90 US 524410
21.09.90 US 586123

(43) Date of publication of application:
05.06.91 Bulletin 91/23

(84) Designated Contracting States:
BE CH DE FR GB IT LI LU NL Bulletin 02/A

(71) Applicant: EASTMAN KODAK COMPANY
343 State Street
Rochester, NY 14650-0221(US)

(72) Inventor: Columbus, Richard Lewis, c/o
EASTMAN KODAK COMPANY
Patent Department, 343 State Street
Rochester, New York 14650-2201(US)
Inventor: Palmer, Harvey John, c/o EASTMAN
KODAK COMPANY
Patent Department, 343 State Street
Rochester, New York 14650-2201(US)

(74) Representative: Phillips, Margaret Dawn et al
Kodak Limited Patent Department Headstone
Drive
Harrow, Middlesex HA1 4TY(GB)

(54) **Blood collection device.**

(57) A blood collection device is well known in which a sample is collected and then centrifuged to separate it into two phases. This device operates on the principle that after centrifuging, and on increasing the centrifugal force applied to the device, a valve opens to allow the lighter phase material to be removed from the collection chamber. However, in these devices, once the valve opens, it stays open as long as the higher centrifugal force is applied. This will allow some of the heavier phase to leave the chamber thereby causing remixing of the two separated phases. Described herein is a blood collection device (30) which prevents the occurrence of remixing and also uses much lower centrifugal forces (CF). The device (30) comprises a separation chamber (32) arranged so that its long dimension ($\ell$) is parallel, not perpendicular, to the spin axis (76) during centrifuging. A valve (50) allows automatic removal of the lighter phase(s) by opening only once a certain head of liquid pressure has been generated by an increased centrifugal force. As a result, whole blood can be readily separated into two or more phases, the lighter phase(s) being extractable without any substantial remixing with the heavier phase.

EP 0 430 356 A2

## BLOOD COLLECTION DEVICE

The present invention relates to devices for separating a light phase from a heavier phase in a multi-phase liquid, particularly whole blood.

Blood collection and separating devices have from time immemorial, spun down the whole blood in a container having its long axis oriented parallel, or mostly parallel, to the direction of the centrifugal force. Examples of such devices are disclosed in US-A-4012325.

There are several reasons for having the container at this orientation. One reason is that when centrifugal forces cease, there is a substantial distance of separation between the heavier red cells and the lighter serum, and at the same time, an interface is formed between the two phases of reduced surface area. As a result, when serum is drawn off, there is less likelihood that the blood cells will redisperse into the lighter serum phase. To further prevent this undesired event, a gel of intermediate specific gravity is often used, to occupy the surface area between the two phases. The spinning of the container about the long axis ensures that the depth of the gel which resists remixing after centrifuging, will be substantial.

Stated from an opposite point of view, it has not been considered feasible to spin such containers about one of the shorter axes. The reason is that the distance between the free surface of the separated serum, and its interface with the separated blood cells, becomes very short, with a concommittant large surface area at the interface. This in turn makes blood cell contamination of the serum as it is "poured off" or removed, more likely. Any attempt to use a gel to shore up such a large surface area interface is less likely to succeed, since the gel will have only a short depth to it to resist remixing. (The volume of the gel will be distributed primarily over that large surface area of the interface.)

However, the conventional approach has paid a price for these conclusions. The price is, that phase separation takes a long time since it has to occur over the longest dimension of the liquid volume. For example, in a blood volume of about 2ml, and using a device similar to that described in US-A-4012325, the time for separation of the serum from the blood cells is on the order of 5.3min when spinning at, for example 100g. It is true, of course, that such separation times are also a function of the centrifugal force applied - the greater the force created by using higher rpm values, the faster the separation. Thus, typically the forces that are used are well in excess of 1000g as lower forces will cause unacceptable delay in the phase separation. But even at such higher forces, such as 1600g, the separation in a 2ml volume container has not been generally possible in less than 30 sec. Most importantly, however, is a disadvantage which has now been discovered about such centrifugal forces, namely, that a layer called the "buffy coat" is formed at the interface between the blood cells and the serum. Among other things, when formed at centrifugal forces in excess of 100g, the buffy coat has as an inseparable part thereof, leukocyte cells such as the lymphocyte cells, which contain useful DNA. If those cells could be drawn off, the DNA could be extracted. The problem has been that the phase separation which occurs using conventional containers and centrifuges therefor, ensures that those lymphocyte cells are irretrievably mixed with the rest of the buffy coat. It will be readily apparent, therefore, that any attempt to speed up phase separation to less than 60s by drastically boosting the force of spinning, will completely interfere with the retrieval of the lymphocyte cells.

Therefore, prior to this invention there has been a substantial need for a blood phase separation device which can be spun about one of its short axes to allow faster phase separation and/or lower spinning forces, while at the same time somehow solving the high risk of remixing of the phases as noted above.

One approach to dealing with this need would be, of course, the provision of some mechanism which allows for ready withdrawal of the light serum phase from the container before the centrifugal force is removed. This in turn will aid in retaining the unwanted blood cells in a capture zone of the container, during serum removal, since the centrifugal force will still be applied. In fact, a blood separator device has been proposed which allows serum removal from the container while spinning still occurs - it even occurs by increasing the spinning speed. The device in question is described in Japanese Kokai 63/237368. A valve is provided in the device which closes off an exit passageway from the container, the valve being spring-biased so that it will open only when the centrifugal force is increased beyond the speed used during phase separation, for example from 3000 to 5000rpm. Clearly, in such a device serum can be drawn off with a minimum of risk of red cells remixing with the serum. However, even in such a device, it was not considered that the "while - centrifuging" serum withdrawal would permit re-orienting the device to spin about its short axis. Instead, the device still requires the conventional spin orientation wherein the phase separation must occur over the long axis of the container.

Another disadvantage of the device shown in Japanese Kokai 63/237368 is that the valve will stay open as long as a high centrifugal force is applied, even in the absence of liquid flow. Clearly, a better construction is one in which the valve automatically closes after all serum is removed. The reasons are that a) failure to do so makes it possible that non-serum components, if somehow loosened in the container, can also get out the valve, and b) the still-open valve prevents other processing from being accomplished on the blood cells remaining in the container while the device is spinning. These disadvantages stem from the fact that the valve of this device operates only in response to centrifugal force, and not in response to the presence of liquid, for example serum, which is to be drawn off.

It is therefore an object of the present invention to provide a two-phase liquid separation device which will more promptly, and at slower speeds, achieve phase separation and automatic removal of the lighter phase, particularly when processing whole blood.

In accordance with one aspect of the present invention, there is provided a liquid phase separation device for separating a liquid into a heavier phase portion and a lighter phase portion using centrifuging, the device comprising:-

a first chamber having a predetermined volume, a longest dimension, and at least one shorter dimension, the chamber having a heavier phase collecting portion and a lighter phase collecting portion;

inlet means through which liquid can be introduced into the chamber;

a second chamber in which the separated lighter phase portion is collected; and

valve means intermediate the first chamber and the second chamber for allowing the removal of the separated lighter phase portion from the first chamber after separation;

characterized in that the heavier phase collecting portion and the lighter phase collecting portion are disposed so that the longest dimension of the first chamber is generally equal to the length of at least one of the collecting portions, and in that the one shorter dimension extends from the lighter phase collecting portion into the heavier phase collecting portion.

By this arrangement, the device is spun about one of the short dimensions of the chamber rather than the long one, and by a more judicious use of valve means, allows the removal of the lighter phase during centrifugation.

In its preferred form, the valve means are responsive only to pressure from the lighter phase, and not to the centrifugal force. The result is a dramatic reduction in forces used for phase separation, to levels which allow recovery of cellular fractions heretofore lost, without extending the total time of centrifugation unreasonably.

Using a device according to the present invention, phase separation for a liquid volume of 500$\mu$l can occur for a spin radius of about 2.5cm, in less than 2 minutes using a centrifuging force no greater than about 30g.

It is an advantageous feature of the invention that a phase separation device and method are provided which give separations of phases such as in whole blood, at drastically reduced centrifugal forces which still require about the same centrifuging times as conventional devices using forces which are hundreds of g greater.

It is a related advantageous feature of this invention that phase separation by centrifugation can be done under low force conditions that allow the recovery of lymphocytes from the cell fraction that is normally lost.

It is another advantageous feature of the invention that such a device and phase separation are provided with valve means which draw off the desired phase while centrifuging is still occurring, only in response to the pressure generated by the liquid to be drawn off.

The present invention will now be described by way of example only, with reference to the accompanying drawings in which:-

Figure 1 is a sectioned elevational view of a known serum separation device;

Figure 2A is a sectioned elevational view of a serum separation device constructed in accordance with the present invention;

Figure 2B is a sectioned view taken generally along the line IIB-IIB of Figure 2A;

Figure 3 is a plot of serum separation time against centrifugal force obtained using the device shown in Figures 2A and 2B;

Figure 4 is a graph showing a plot of recovered lymphocytes versus the centrifugal force used for phase separation;

Figure 5 is a sectioned elevational view similar to that shown in Figure 2A, but illustrating a second embodiment of a serum separation device constructed in accordance with the present invention;

Figures 6A and 6B are fragmentary sectioned views showing two operating positions of another valve arrangement which could be used in the device shown in Figure 5;

Figure 7 is an elevational view of a further embodiment of a separation device constructed in accordance with the present invention;

Figures 8 to 10 are fragmentary sectioned views taken generally along the respective lines VIII-VIII, IX-IX, and X-X of Figure 7;

Figure 11 is a sectioned elevational view taken generally along the line XI-XI of Figure 9 of the device shown in Figure 7;

Figure 12 is an enlarged, fragmentary, partially schematic perspective view of the capillary zone in each of the chambers shown in Figure 9;

Figures 13 and 14 are sectioned views similar to that shown in Figure 11, and illustrates the fill sequence of the device shown in Figure 7;

Figures 15 and 16 illustrate the liquid configurations after phase separation and then transfer of serum past the valve respectively; and

Figures 17 and 18 are fragmentary elevational views similar to those shown in Figures 2 and 5, but of a still further embodiment of a device constructed in accordance with the present invention.

The invention is hereinafter described in light of its use in preferred embodiments, wherein blood serum or plasma is the lighter phase of a two-phase liquid, and particularly preferred chambers are described for collecting the serum and/or lymphocytes valved off from the two separated phases, using a ball check valve. In addition, the invention is useful regardless of the multiple-phase liquid it is used with, regardless of the type or even presence of a subsequent chamber downstream of the valve, and regardless of the valve construction; so long as the valve that is used meets the requirements of the invention.

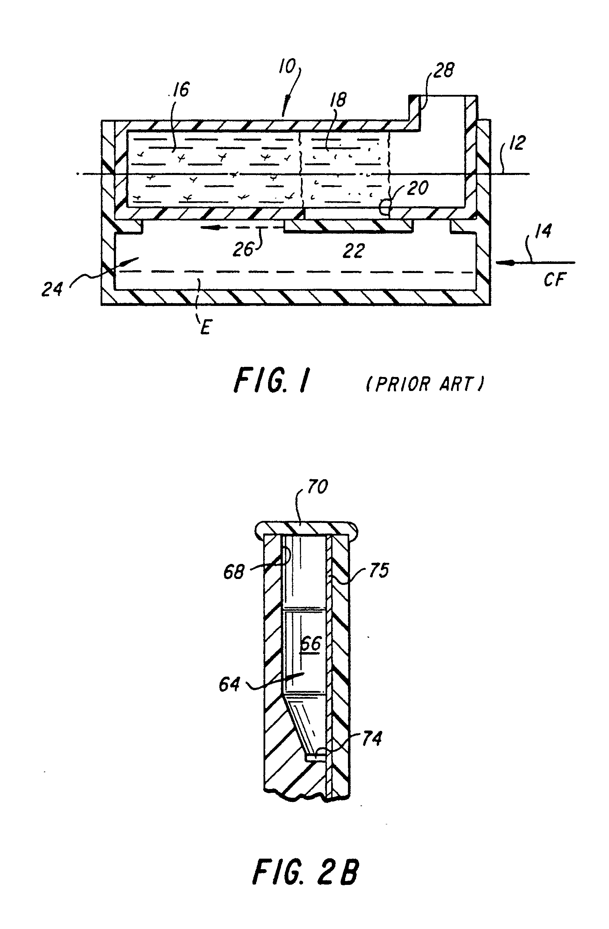

Many known serum separators have conventionally used a container 10, as shown in Figure 1, in which the longitudinal axis 12 of the container is parallel to the direction of centrifugal force CF, as indicated by arrow 14. As a result, substantial time and force is required to separate the heavier blood cells 16 from the lighter serum 18 into the two fractions shown. In some devices, for example in the device disclosed in Japanese Kokai 63/237368 noted above, an aperture 20 is provided along with a valve 22 to allow the serum to flow into a separate-like chamber 24 where it can contact a slide-like test element E, which is shown in phantom. Valve 22 is constructed to open only when a centrifugal force greater than that used to separate the two phases is achieved, and then the valve moves in the direction of arrow 26 against a return spring (not shown) to open aperture 20. This construction has all the attendant disadvantages noted above. In addition, whole blood is added to the device by pouring it through aperture 28. This requires operator attention or an intermediate machine step after whole blood is collected in a separate operation using a needle.

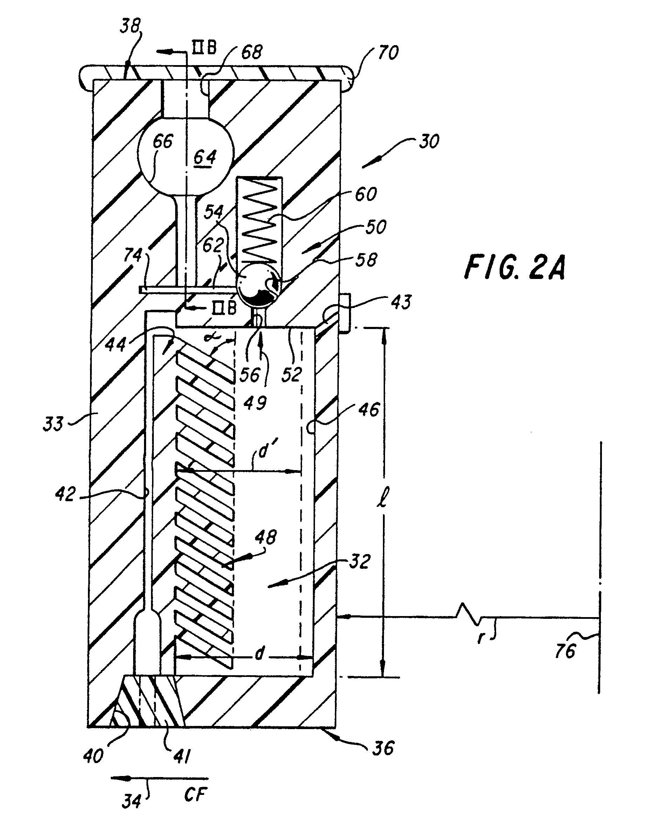

In accordance with the present invention, a phase separation device 30 is shown in Figure 2A, The device 30 allows for phase separation of at least two phases. It comprises a phase separation chamber 32 which has its long dimension $\ell$ oriented perpendicular, not parallel, to the direction of centrifugal force CF, shown by arrow 34. It also has a specially constructed valve 50. Chamber 32 is defined by a body member 33 which has a blood intake port 36 at one end and a serum removal port 38 at the other end as shown. Chamber 32 extends from port 36 to delivery passageway 56. Port 36 has an aperture 40 through which blood is introduced into the device 30. The aperture 40 is filled with a conventional septum 41, and chamber 32 is either vented at 43 or evacuated due to attachment at 43 to an external vacuum source (not shown) to assist in blood intake.

Aperture 40 allows entrance of whole blood via passageway 42 to chamber 32. The width d of chamber 32 is one of the shorter dimensions, enough blood being drawn in to fill to about the depth d'. Sidewall 44 of chamber 32 is the sidewall against which the heavier blood cells collect, whereas opposite sidewall 46 is adjacent the lighter serum fraction during centrifugation. Thus, dimensions d and d' extend from the lighter phase into the heavier phase.

Optionally, fixed porous mechanical means, such as baffles 48, can be positioned along wall 44 so as to be disposed in the blood cells. As described in co-pending European patent application no. 90 302 703.5, such means act to prevent the heavier phase from remixing when the lighter serum phase as it is drawn off from the phase separating container. The plates of the baffles are inclined at an angle $\alpha$ which resists remixing forces when flow occurs out of chamber 32 in the direction of arrow 49. Preferably, this angle is a value which is between about 30° and about 120°, most preferably about 60°. Preferably, the distance between the individual plates of baffles 48 is between about 0.018cm and about 0.10cm, most preferably about 0.025cm. The thickness of each plate is not critical, so long as a significant number of such plates are present as will create the needed volume between them to collect the blood cells.

Valve 50 is disposed at an end 52 of chamber 32 intermediate ends 36 and 38, and is positioned to draw off separated serum or plasma and lymphocytes (discussed hereinafter). Most importantly, valve 50 is constructed to open only in response to a hydraulic head of force, and not to the effects of centrifugal force CF regardless of the magnitude of the latter. To this end, valve 50 is preferably a ball check valve with a ball 54 positioned downstream of passageway 56 at chamber end 52. Ball 54 seats against a hemispherical seat 58, and is biased by a spring 60 aligned to act in a direction which is generally perpendicular to the direction of centrifugal force CF. This alignment tends to ensure that ball 54 will act against spring 60 only in response to forces other than centrifugal force CF.

A serum or plasma exit passageway 62 is constructed adjacent seat 58 to allow liquid to be drawn off when valve 50 opens. Passageway 62 joins a chamber or compartment 64 sized to receive substantially all the liquid which exits in chamber 32 via valve 50. Chamber 64 has a deep well portion 66 designed to collect lymphocytes, and a large opening 68 adapted to allow a pipette access to chamber 64 generally and to well portion 66 in particular. A cover 70 is removably sealed over opening 68 except when access of the pipette or other removal means is desired.

Passageway 62 preferably extends beyond chamber 64 to a trap 74. The function of the trap 74 is to collect the few red blood cells which may gather prior to and during centrifuging in passageway 56. This allows only desired serum, or plasma and lymphocytes to pass into chamber 64.

A vent passageway 77 is preferably provided under seal 70 to vent entrapped air as serum is transferred into chamber 64.

Device 30 can be assembled as two plates, as shown in Figure 2B, using a foil layer 75 to achieve a seal which will allow a vacuum to be drawn using vent 43, as described above.

Such a device 30 can be spun in any convenient centrifuge (not shown) where the long dimension $\ell$ is generally parallel to the spin axis 76 (as shown in Figure 2A). Preferred spin radii are about 2.5cm, although a wide variety can be used.

To prevent air entrapment, particularly where such may impede incoming liquid flow, various vents are provided. For example, vent 77 is preferably provided for chamber 64 as shown in Figure 2A. Additionally, the baffle plates of baffles 48 can have ribs (not shown) which allow entrapped air to escape.

The method of phase separating using device 30, will now be described. Whole blood is placed into chamber 32 using a needle, for example, which penetrates septum 41. Device 30 is then spun about axis 76. However, in accordance with another aspect of the present invention, the speed of rotation which is selected is slow - a speed producing no greater than 400g centrifugal force, and most preferably no greater than 30g. The reason is that device 30 is capable of achieving phase separation at such forces, using 2ml of liquid, in less than 2min, and in some cases less than 1min, due to the (relatively) short distance (about d'/2) which the blood cells have to traverse to be separated.

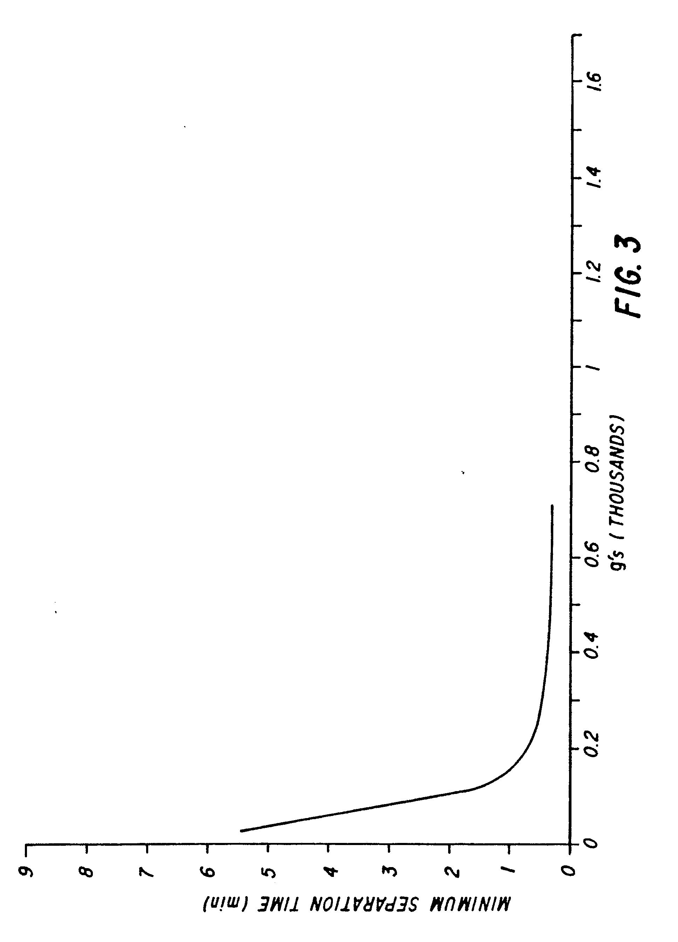

Figure 3 illustrates the separation times achievable with the invention, using a 2.5cm spin radius and a total whole blood volume of $500\mu L$. As indicated, the serum, or plasma and lymphocytes, can be separated in less than 1min if the centrifugal force is about 150g or greater, there being little separation time enhancement occurring at forces above 400g. At the other end, a separation force of only 30g will produce complete phase separation in less than 8min, for example, 5.5min. As a comparative example, as described in US-A-4818418, the conditions achieved using a conventional Ficol-Pague/Percoll as an additive were found to be effective to achieve separation only after 30 minutes when a centrifugal force of 400g is applied.

Whatever centrifugal force is selected, after serum or plasma separation occurs the lighter phase is then drawn off the stacked liquid in chamber 32 by opening valve 50. This occurs as follows: spring 60 has a spring constant $K_1$ which is pre-selected to resist movement of ball 54 until a certain head of pressure builds up against ball 54. The increased head of pressure occurs by increasing the centrifugal force a factor, for example 50%, above the force used to achieve phase separation. Preferably, the speed of rotation is increased a corresponding amount. Since the serum and blood cells are relatively incompressible against wall 44, the increase in centrifugal force CF translates into an increased force in the direction of arrow 49, which overcomes spring constant $K_1$ of spring 60, and the valve opens. However, this is true only as long as enough serum or plasma remains in chamber 32 to push out aperture 56. When most of the serum or plasma has passed through the valve, the head of pressure occurring even at the increased speed of rotation, drops. As a result, valve 50 closes automatically even at the higher speeds of rotation, unlike the operation of valve 22 discussed in relation to Figure 1.

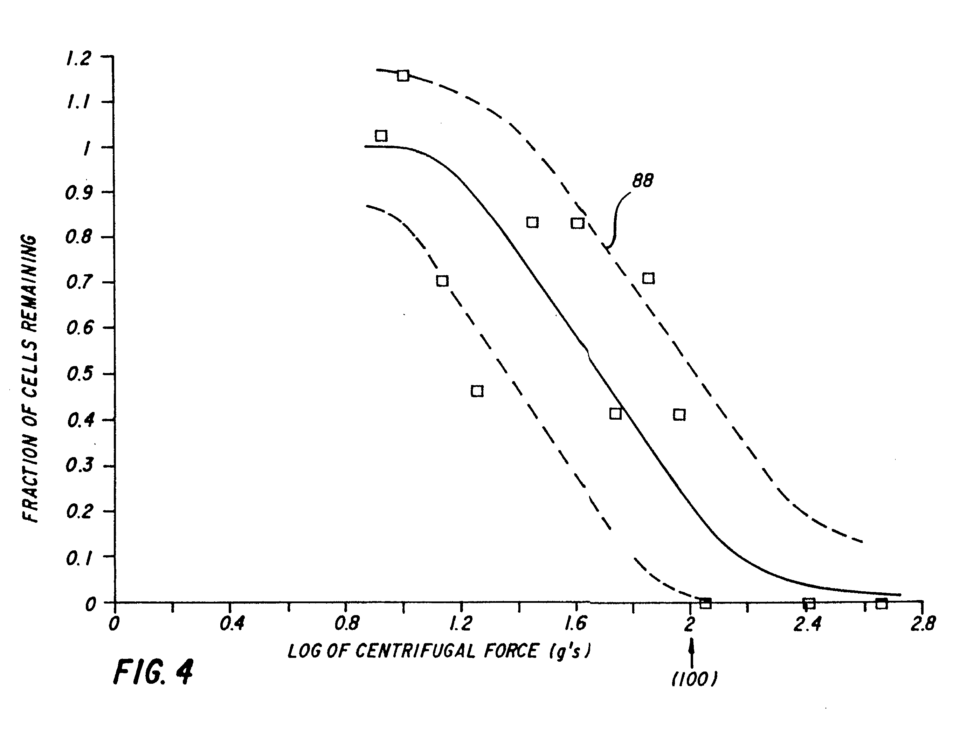

Figure 4 illustrates that in fact this process does produce the separation of lymphocytes, without the necessity of using a chemical phase separation agent common in conventional lymphocyte separation by centrifuging. (If lymphocytes are the desired end-product, then plasma is the lighter phase, rather than serum. Serum is the same as plasma, except that in serum the fibrinogen has been removed, a step considered detrimental to obtaining lymphocytes.) Because the centrifugal forces are at a level below about 100g, the lymphocytes do not get irretrievably compacted into the buffy coat, as is the case in known centrifuges which operate at forces above 100g.

More specifically, the graph of Figure 4 was prepared using a device of the type shown in Figure 2A, in a centrifuge rotor where r has the value 2.54cm (1in). Since lymphocytes can all be lost in the red cells if

5

the spin time is allowed to proceed too long before opening valve 50, it is necessary that the process be sampled at varying times for any given centrifugal force $G_i$. Thus, for, for example $CF = 50g$, many spin times (between 1 and 10min) were examined to determine what optimal time for that centrifugal force CF would produce the maximum amount of lymphocytes remaining in the plasma. This is the same as the amount transferred by opening valve 50 by increasing the force CF. The amount of the lymphocytes so remaining in the lighter phase at those optimized times, for each different g force, was then plotted versus the g forces, as a fraction of the total original amount of lymphocytes, expressed as a log to the base 10.

Figure 4 illustrates the results. A band 88 surrounding the curve has been drawn to "fit" the data. This band symbolizes the uncertainty in the data, where each data point is the mean for the tests. No standard deviation has been determined, however. As noted, the important feature is the recovery of significant fractions of the lymphocytes available. This occurred where the centrifugal force was less than 100g.

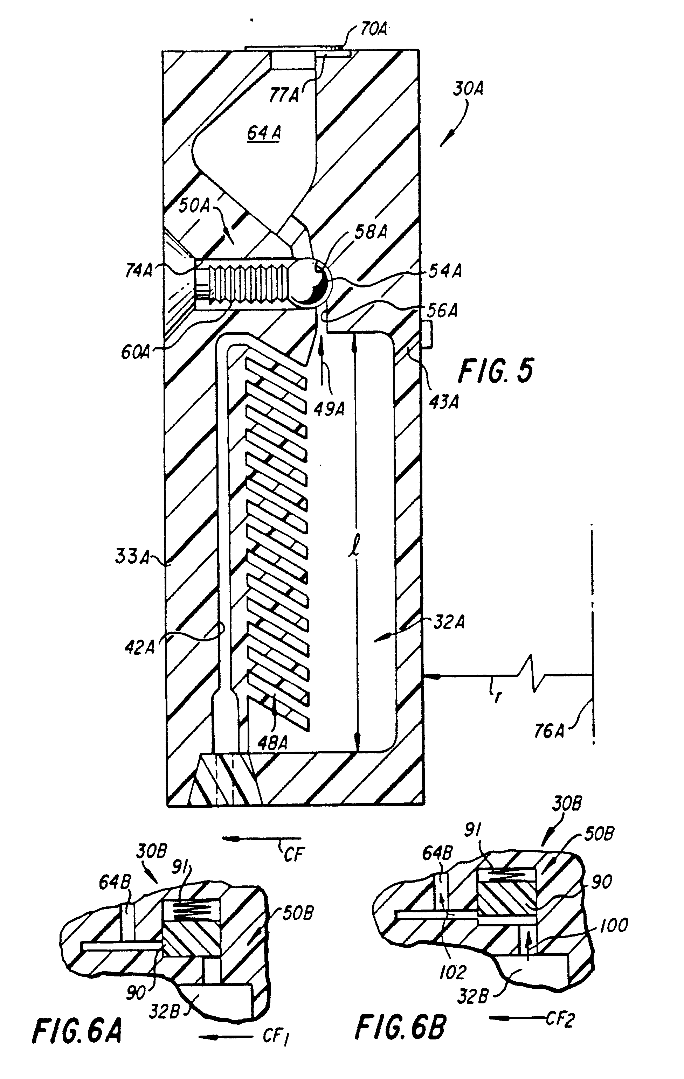

It is not essential that the valve described above operates on an axis which is neutral to the centrifugal force. Another valve embodiment is shown in Figure 5. Parts similar to those previously described bear the same reference numeral, to which the distinguishing suffix "A" has been appended.

Thus, device 30A comprises a body 33A having a chamber 32A, passageway 42A supplying blood thereto as before. Baffles 48A can be included to retain the heavier blood cells, and passageway 56A allows removal of the lighter phases such as lymphocytes and plasma, from chamber 32A into covered chamber 64A, using valve 60A. The long dimension $\ell$ of chamber 32A is parallel to spin axis 76A. However, in this embodiment spring 60A is oriented to be parallel to the direction of centrifugal force CF. Nevertheless, the spring constant $K_2$ of spring 60A is selected so that ball 54A still opens only in response to a liquid head of pressure, and not in response to the centrifugal force. When ball 54A lifts off seat 58A, the lighter phases pour into chamber 64A. In this embodiment, the volume of passageway 74A which is not filled by spring 60A is just enough to trap any blood cells caught in passageway 56A prior to phase separation.

The careful selection of spring constant $K_2$ of spring 60A is as follows: It is selected so that valve 50A will not open at the first centrifugal speed $CF_1$ used to achieve phase separation. Moreover, it is strong enough to prevent valve opening even in the presence of the higher centrifugal speed $CF_2$ used to create a head of pressure on the valve, in the absence of any liquid pressing on ball 54A. However, because ball 54A has a surface which is included at a non-90° angle to the force of arrow 49A, ball 54A will incur a force parallel to $CF_2$ due to a liquid head of pressure $\Delta P$ generated in the direction of arrow 49A, caused by centrifugal force $CF_2$. (The component of $\Delta P$ which is parallel to $CF_2$ is hereinafter designated $\Delta P_{CF}$.) Therefore, spring constant $K_2$ is greater than the force generated by $CF_2$ alone, but less than $(CF_2 + \Delta P_{CF})$. When all of the lighter phase liquid has transferred to chamber 64A, there no longer is a liquid head of pressure creating a force $\Delta P_{CF}$, and valve 50A closes automatically, even in the face of a centrifugal force $CF_2$.

The contents of chamber 64A, such as lymphocytes and plasma, are then aspirated out, by removing cover 70A.

The valve for automatic removal of the lighter phase need not be a ball valve, to respond only to the liquid head of pressure. Any valve can be used, if it is constructed to resist forces other than this head of pressure. Another type is shown in Figures 6A and 6B, in which parts similar to those previously described bear the same reference numeral, to which the distinguishing suffix "B" is appended.

Thus, device 30B includes blood collection and separation chambers such as chamber 32B, and a valve 50B which operates only in response to a head of liquid pressure to pass the lighter phase into separate chamber 64B, as in the previous embodiments. However, whereas the previous valves used balls, valve 50B comprises a solid rectangular block 90 backed by a spring 91 of a suitable spring constant selected to deform enough to open the valve, only when centrifugal force is increased from $CF_1$ as shown in Figure 6A, to $CF_2$ as shown in Figure 6B. Flow then proceeds through the open valve as shown by arrows 100, 102.

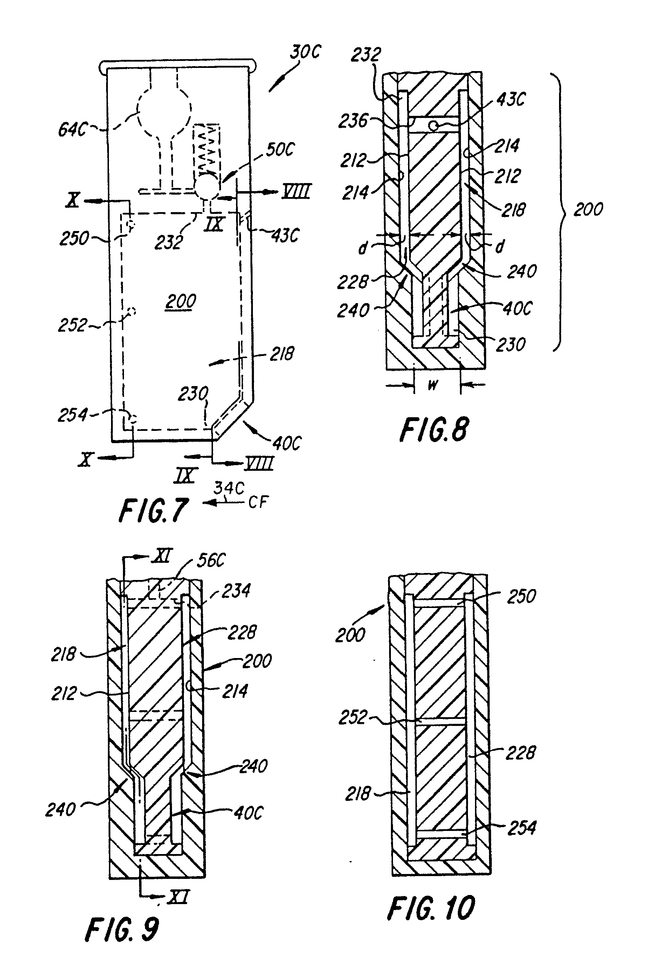

The above embodiments are all directed at a device according to the present invention constructed for use with a phlebotomy syringe. In addition, the device according to the present invention can be used to collect and process blood from a finger prick, using capillary attraction forces to draw in the blood as shown in Figures 7 to 16. Parts similar to those previously described bear the same reference numeral, to which the distinguishing suffix "C" is appended.

Device 30C is substantially the same as the previous embodiments from the valve 50C downstream to the serum chamber 64C. Upstream, however, as shown in Figure 7, it is constructed at portion 200 with at least one capillary chamber 218 having opposing walls 212 and 214, as is shown more clearly in Figure 8. The walls 212, 214 are spaced apart a capillary distance d to cause capillary attraction to draw in liquid. An inlet aperture 40C allows pooled blood, for example from a finger prick, to access chamber 218. Thus, portion 200 is similar to the finger prick, capillary attraction collection and separation device described in US-A-4136036. Once whole blood fills chamber 218, the device can be spun about an exterior axis to generate a centrifugal force CF, indicated by arrow 34C in Figure 7, to achieve serum and blood cell separation in chamber 218. Serum can then be transferred past valve 50C and into serum chamber 64C exactly as is described for previous embodiments.

Because capillary spacing d (shown more clearly in Figure 8) does not provide for much overall collected volume, it is preferred that more than one capillary collection chamber be present. Accordingly, at least a second chamber 228, and optionally even a third (not shown) is disposed adjacent chamber 218. Both chambers have a proximal end 230 and a distal end 232. In each case, end 230 is fluidly connected to inlet aperture 40C, while end 232 is fluidly connected via perpendicular passageway 234 to transfer passageway 56C as shown in Figure 9. Thus, chambers 218 and 228 are disposed to act in parallel, to simultaneously collect by capillary attraction, whole blood touched to inlet aperture 40C.

An air vent 43C is provided (see Figures 7 and 8) as in the previous embodiment to vent entrapped air. Each chamber is directly connected to vent 43C by a perpendicular passage 236 (Figure 8) disposed immediately adjacent vent 43C.

To bring the chambers closer together at aperture 40C, in light of the small volumes and dimensions characteristic of a finger prick, major walls 212 and 214 of each chamber preferably are beveled at 240 adjacent to aperture 40C, as shown in Figures 8 and 9. This minimizes the width w (see Figure 8) which is required.

To equalize the liquid volumes which might build up in one of the two parallel chambers due to the other filling faster, passageways 250, 252 and 254 are provided, as shown in Figures 7 and 10, to fluidly connect together all of the chambers of portion 200. These passageways are preferably disposed in between proximal end 230 and distal end 232.

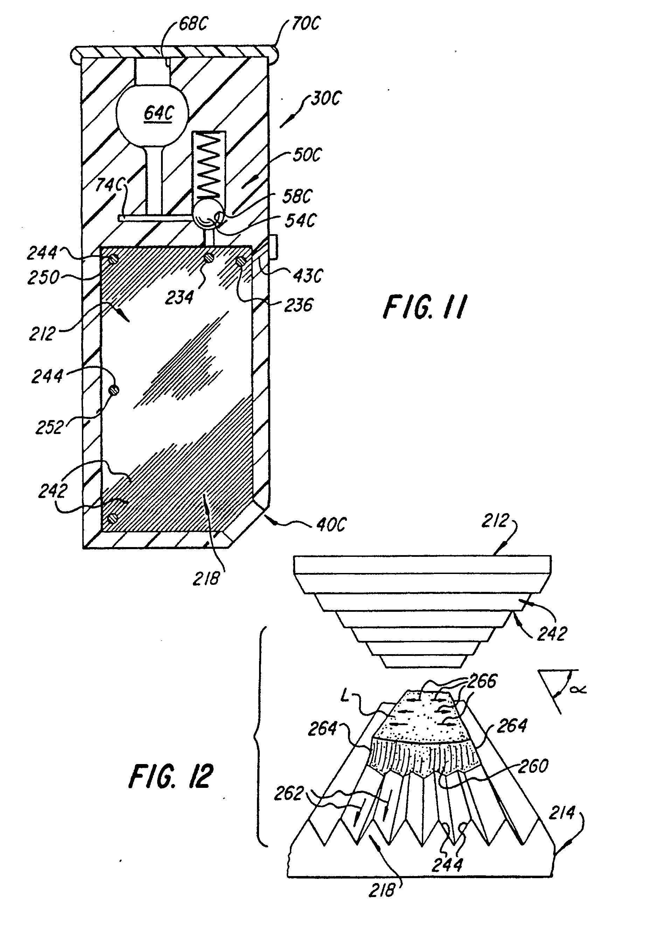

The surfaces of walls 212 and 214 can be nominally smooth. Preferably, however, they are provided with grooves 242 on wall 212 and 244 on wall 214, as shown in Figures 11 and 12. The grooves are also preferably positioned and formed so that grooves 242 are disposed at an angle $\alpha$ to grooves 244 (Figure 12). The purpose of these angled grooves is to provide control of the waveform of the advancing menisci 260, 264, as described in US-A-4233029. Thus, menisci 260 and 264 will advance, in the directions indicated by arrows 262 and 266, with the shape of the grooves, which for linear grooves as shown will produce a trapezoidal waveform which will be rectangular if angle $\alpha$ is $90°$. This control of the waveform further ensures that there will be no entrapped air bubbles and that chambers 218 and 228 will completely fill with whole blood. Furthermore, hysteresis caused by the grooves ensures that the intake of whole blood from a wound can be interrupted without the trailing meniscus advancing so far into aperture 40C that an air bubble is formed when intake is resumed.

Although only one opposing wall grooves 242 are visible in Figure 12, grooves 244 of the other opposing wall for the chamber 228 can be seen through passageways 250, 252, 254 and connecting passageway 234.

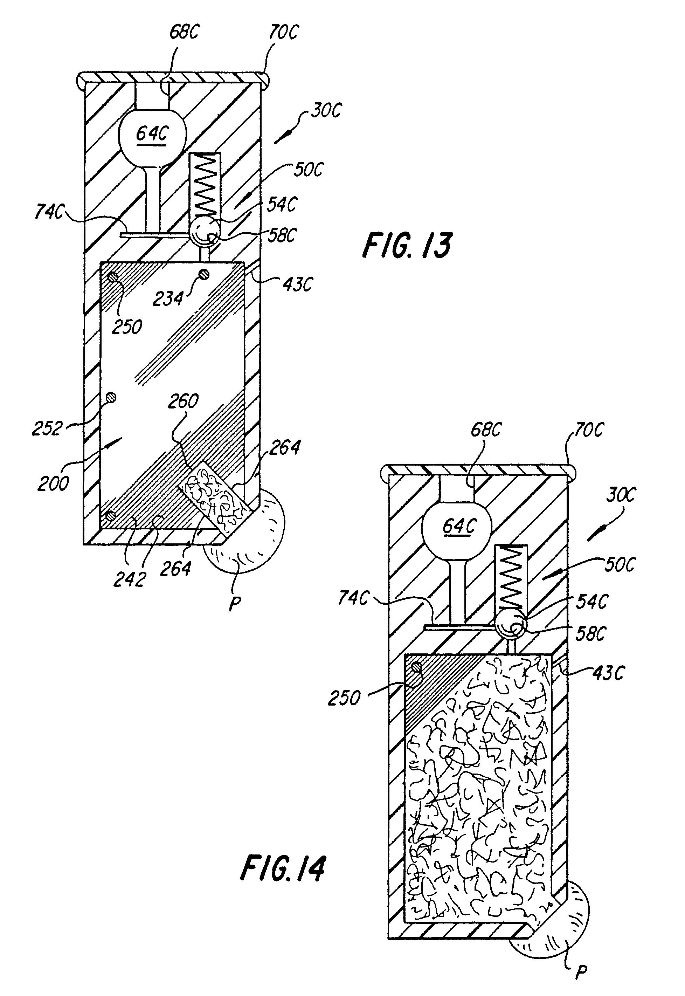

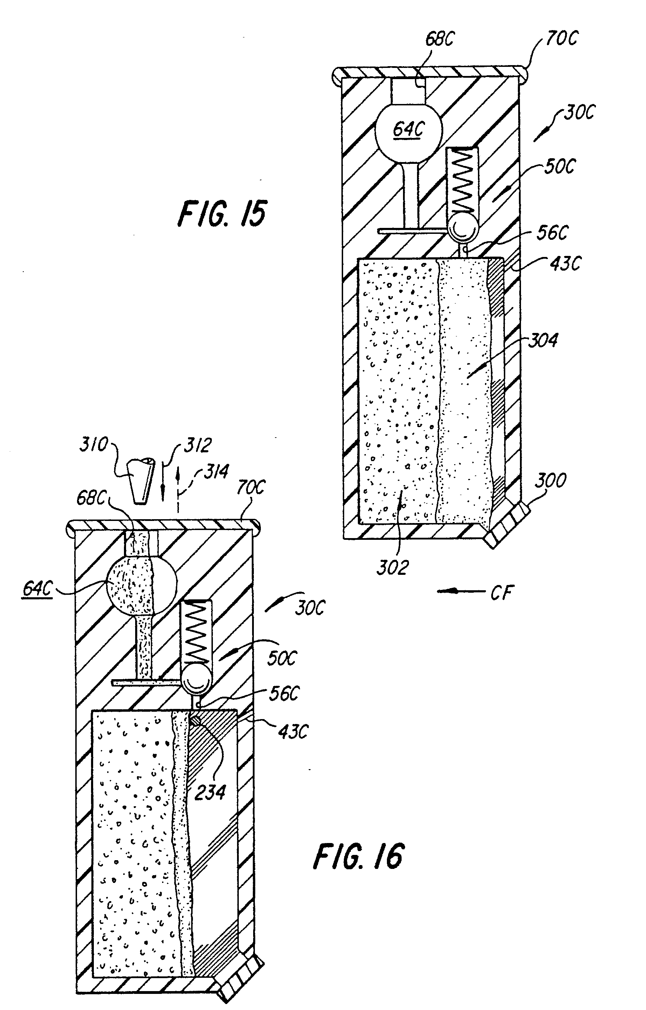

The operation of device 30C will now be described with reference to Figures 13 to 16. When the device 30C is touched to a pool P of blood (Figure 13) the whole blood advances at portion 200 to simultaneously fill both chambers 218 (and 228, not shown), with menisci waveforms 260 and 264 dictated by the linearity or other shape of grooves 242 (and 244, not shown). The advance continues, as shown in Figure 14, with passageway 250 being the last to contact the blood. Once chambers 218 and 228 are filled, aperture 40C is capped by any suitable cover 300 (Figure 15) and the device is spun to produce a centrifugal force of about 400g. This separates the blood into its two phases. The circles of phase 302 indicate blood cells, whereas the dots of phase 304 indicate serum. Thereafter, force CF is increased to cause hydrostatic pressure of the serum against valve 50C, and transfer of serum occurs into chamber 64C via passageway 56C. After centrifuging, the serum can be easily removed from chamber 64C by penetrating seal 70C with a pipette 310 (Figure 16). The pipette 310 is inserted in the direction of arrow 312, and the serum withdrawn in the direction of arrow 314. (Aperture 68C is sized to allow access of the pipette).

Still another use that can be made of chamber 64 (not shown) is as one of the twin receptacles used to fill a dual pipette. The twin receptacles or tubs conventionally used for filling a dual pipette, comprise one in which a reference liquid is pre-inserted (or subsequently added) while the other one is chamber 64 into which serum is transferred when the valve 50C is opened.

The valve between the two chambers need not be a ball and spring, and indeed, a smaller, less expensive valve can be constructed using the embodiment shown in Figures 17 and 18. Parts similar to those previously described bear the same reference numeral, to which the distinguishing suffix D or E has been appended, respectively.

Thus, in Figure 17, the device 30D has chambers 32D and 64D connected by passageway 56D and 62D, to function exactly as described above for the embodiment of Figure 2A. However, valve 50D comprises a biasing member 60D and a closure member 54D which seats against valve seat 58D, wherein members 60D and 54D are selected from materials different than those illustrated heretofore.

More specifically, the biasing member is preferably a cellular foam having a Young's modulus of no larger than about 345kPa so as to be readily compressed using the forces described above. In addition, however, it should most preferably permit the valve to open as required under compressions (arrow 400) which are no greater than 40%, to avoid buckling the valve.

Most preferably, such foam is an open-celled foam, of which polyurethane foam is a highly preferred example, albeit foams of other polymers which are inert to blood, or whatever fluid is being transferred, can be used.

A specific example of such polyurethane foam which has been shown to be effective is such foam available under the tradename "Poron", from Rogers Corporation. Of the "Poron" products, "Poron 4701-01" is particularly useful. This material has the following typical range of physical properties, as shown by the Rogers Corp. Physics Properties Data Sheet dated 1989:

| Property | Test Method | Range | |
|---|---|---|---|
| Density +/− 10% (kg/m3) | ASTM 3574 | 240 | 320 |
| Color (Code) | Black | Black | |
| Compression Set | ASTM 1667 @ 73°F (23°C) | <2% | <2% |
| | ASTM 3574 @ 158°F (70°C) | <10% | <10% |
| | ASTM 3574 after 5 hours @ 250°F (121°C) | <5% | <5% |
| Compression Force Deflection (Young's Modulus) (kPa) | 0.2"/min Strain Rate Force measured @ 25% Deflection | 42 ± 14 | 69 ± 21 |
| Durometer | Shore "O" | 12 | 17 |
| Tensile Strength min. (kPa) | @ 20"/min Strain Rate | 275 | 620 |
| Elongation % min | @ 51 cm per min | 100 | 100 |
| Temperature Resistance | | | |
| Recommended Constant Use | 70°C | 70°C | |
| Intermittent | 121°C | 121°C | |
| Cold Flexibility | MIL−P−12420C @ −40°C | Pass | Pass |
| Embrittlement Temperature | ASTM D746 | −40°C | −40°C |
| Flame Spread | MVS S302 | Pass | Pass |
| Thickness min. (mm) | | 3.2 | 3.2 |
| Outgassing % Total Mass Loss | ASTM E−595 | 1.30 | 1.30 |
| Thickness Tolerance | ±10% | ±10% | |
| Capabilities (mm) | | 2.5−12.7 | 1.6−12.7 |
| Thermal Conductivity BTU/(HrFt2)/(°F/in) | "K" Factor | 0.5 | 0.5 |
| Coefficient of Thermal Expansion | | 1.3−1.8 x 10−4/°C | |
| Corrosion Resistance | AMS 3568 | Excellent | |

Regarding closure member 54D, it is made from an impervious material which by its flexible nature and thickness must be such as will conform to and seal against valve seat 58D, without sticking, when it is biased into contact with the seat. As used herein, "conform" means, to generally follow the shape and configuration of that seat, which here is shown as being a generally flat surface. However, other surfaces also can be used. Also as used herein, "without sticking" means, without adherence of the closure member on the valve seat at the time of use such as will significantly increase the force required to displace the closure member from the seat, that is by more than 5% compared to the force required at the time of assembly of the valve in the device. Such sticking is particularly evident with many forms of rubber, after several weeks of storage of the device prior to use, especially if stored at elevated temperatures.

In addition, the material of the closure member preferably is also dimensionally stable, that is, it is a material which is relatively resistant to cold flow.

Most preferably, the materials found to provide such non-sticking, imperviousness, flexibility and conformability, are either a preferred polymer tape; or a metallized polymer tape such as one of the preferred polymer tapes which is metallized with silver metal. The skinning over of the foam which forms biasing member 60D, and most rubbers, have not been found to be acceptable. Materials useful for forming the polymer tapes of closure member 54D include polyolefins and as polyethylene and polypropylene and copolymers, including block copolymers of the same monomers and other modified polyolefins available from duPont, Dow Chemical Co., and others, for example, under the trade-name "Handi-Wrap II". Certain preferred sheet film materials are the halogenated olefin polymers such as polyvinylidene chloride copolymers, for example, poly(vinyl chloride-co-vinylidene chloride) and poly(acrylonitrile-co-vinylidene chloride) (the Saran® resins sold by Dow Chemical Co.), poly(vinyl chloride) (PVC), and poly-(tetrafluoroethylene), e.g., Teflon sold by duPont and copolymers thereof, e.g., poly(hexafluoropropylene-co-tetrafluoroethylene). Acrylic polymer sheet materials are also useful such as poly(methyl methacrylate), poly(methyl acrylate), poly(ethyl methacrylate) and other homo- and copolymers of inert acrylic esters, for example, the Elvacite acrylic resins sold by duPont. A preferred class of materials is the celluloses typically employed as film support materials for photographic, electrophotographic, magnetic tape, and transparent adhesive tape coatings such as cellulose acetate, cellulose triacetate, cellulose acetate butyrate, nitrocellulose, etc. Other preferred polymers include well-known polyesters, polycarbonates and polyamides such as poly(ethylene terephthalate) (PET) sold under the tradename Mylar by duPont and Estar by Eastman Kodak Company, bisphenol A polycarbonates such as the Lexan polycarbonates sold by General Electric Co., and nylon sold by duPont.

Especially preferred materials are those supplied with a pressure-sensitive adhesive coating to allow quick, easy application to biasing member 60D. Examples are poly(ethylene terephthalate) and cellulose acetate films, with or without a matte finish, and containing a pressure-sensitive adhesive, exemplary such films being Scotch Brand Tapes Numbers 483, 810, and 850, sold by 3M Co.

It is understood that tacky, soft, or sticky materials such as rubbers and ionic or other hydrophilic polymers are to be avoided to prevent sticking of the tapes on storage. Thus, the celluloses, polyesters, and halogenated polyolefins are preferred; however, this problem can be minimized or avoided by application of metal coatings on foils to the tape.

Adhesives useful for bonding the closure members lacking a pre-applied adhesive include the polymer resins, rubber cements and mucilages known in the art, for example, pressure-sensitive adhesives as described in US-A-2358761; US-A-2553816 and US-A-2783166; ethylene-vinyl acetate copolymers (EVA resins) such as HA6164 sold by Borden Chemical Company, and Elvax polymers sold by duPont, and diolefin-styrene block copolymers, that is polyisoprene resins such as the Kraton resins sold by Shell Chemical Co. and the Solprene resins sold by Phillips Petroleum Company.

The following polymer tapes were tested and found to be acceptable:

| Material | Available From | Under the Tradename . |
|---|---|---|
| (1) modified polyethylene | Dow | "Handi—Wrap II" |
| (2) cellulose acetate | 3M | "Scotch Brand Tape" No. 810 |
| (3) cellulose acetate | 3M | "Highland Brand Tape" No. 6200 |
| (4) polyethylene | 3M | Scotch Brand Tape" No. 483 |
| (5) polyester | 3M | "Scotch Brand Tape" No. 850 |
| (6) Polytetrafluoroethylene | 3M | PTFE "Teflon" |
| (7) Polycarbonate | General Electric | "Lexan" |

Not all thicknesses of such tapes are sufficiently thin to make them conform as noted. The maximum thicknesses and preferred thicknesses are as follows; for the above-tested tapes:

| Type | Maximum Thickness | Preferred Thickness |
|---|---|---|
| (1) as listed above | 0.15 mm | 0.013 mm |
| (2) as listed above | 0.10 mm | 0.06 mm |
| (3) as listed above | 0.10 mm | 0.06 mm |
| (4) as listed above | 0.20 mm | 0.13 mm |
| (5) as listed above | 0.15 mm | 0.05 mm |
| (6) polyester silvered on one side | 0.15 mm | 0.08 mm |
| (7) as listed above | 0.15 mm | 0.09 mm |
| (8) as listed above | 0.15 mm | 0.03 mm |

The closure member comprising such a material is readily adhered to biasing member 60D by any suitable adhesive

Similarly, Figure 18 illustrates a valve operating in response to pressure transmitted parallel to centrifugal forces, in the manner shown for Figure 5, but using a valve constructed of the materials described for Figure 17. Thus, device 30D has chambers 32E and 64E, connected by passageway 56E within which valve 50E is located. As in Figure 17, valve 50E comprises biasing member 60E pressing closure member 54E against valve seat 58E, with members 60E and 54E being constructed as described for Figure 17.

The valve shown in Figures 17 and 18 need not be used only in a two-phase liquid collection and separation device. In addition, it can be used in controlling liquid flow of any type, between any two locations, particularly where the pressure used to initiate transfer is small, e.g., on the order of about 6.8 to 68kPa. Thus, for example, the valve can be used where small amounts of biological fluids of any kind are sequentially transferred between chambers, with time delays between transfer either for the purpose of separating cellular components from supernatant which require a fixed time, or to provide adequate time to carry out reactions such as binding of soluble components to solid surfaces before the sample is transferred to another chamber.

## Claims

1. A liquid phase separation device (30; 30A; 30B; 30C; 30D; 30E) for separating a liquid into a heavier phase portion and a lighter phase portion using centrifuging, the device comprising:-
   a first chamber (32; 32A; 32B; 218, 228; 32D; 32E) having a predetermined volume, a longest dimension ($\ell$), and at least one shorter dimension (d), the chamber (32; 32A; 32B; 218, 228; 32D; 32E) having a heavier phase collecting portion (44) and a lighter phase collecting portion (46);
   inlet means (40, 41; 40C) through which liquid can be introduced into the chamber (32; 32A; 32B; 218, 228; 32D; 32E);
   a second chamber (64; 64A; 64B; 64C; 64D; 64E) in which the separated lighter phase portion is collected; and
   valve means (50, 54, 58, 60; 50A, 54A, 58A, 60A; 50B, 90, 91; 50C, 54C, 58C, 58C; 50D, 54D, 58D, 60D; 50E, 54E, 58E, 60E) intermediate the first chamber (32; 32A; 32B; 218, 228; 32D; 32E) and the second chamber (64; 64A; 64B; 64C; 64D; 64E) for allowing the removal of the separated lighter phase portion from the first chamber (32; 32A; 32B; 218, 228; 32D; 32E) after separation;
   characterized in that the heavier phase collecting portion (44) and the lighter phase collecting portion (46) are disposed so that the longest dimension ($\ell$) of the first chamber (32; 32A; 32B; 218, 228; 32D; 32E) is generally equal to the length of at least one of the collecting portions (44, 46), and in that the one shorter dimension (d) extends from the lighter phase collecting portion (46) into the heavier phase collecting portion (44).

2. A device according to claim 1, wherein the valve means (50, 54, 58, 60; 50A, 54A, 58A, 60A; 50B, 90, 91; 50C, 54C, 58C, 58C; 50D, 54D, 58D, 60D; 50E, 54E, 58E, 60E) only opens in response to a liquid head of pressure produced in the first chamber (32; 32A; 32B; 218, 228; 32D; 32E).

3. A device according to claim 2, wherein the liquid head of pressure is generated at a certain value of centrifugal force which exceeds that required to produce separation of the liquid.

4. A device according to any one of the preceding claims, further including baffle means (48; 48A) positioned in the first chamber (32; 32A) for restricting remixing of the two phases after separation.

5. A device according to any one of preceding claims, wherein the first chamber comprises two chamber portions (218, 228) disposed adjacent to one another and acting in parallel, the two chamber portions (218, 228) each having a proximal end (230) and a distal end (232) and are fluidly connected to the inlet means (40C) at the proximal end (230) and to the second chamber (64C) at the distal end (232).

6. A device according to claim 5, wherein the chamber portions (218, 228) are defined by opposed surfaces (212, 214) spaced apart a capillary distance so that liquid entering the inlet means (40C) is drawn into the chamber portions (218, 228) by capillary attraction.

7. A device according to claim 6, wherein the opposed surfaces (212, 214) for each of the chamber portions (218, 228) are provided with grooves (242, 244), the grooves of one surface of a chamber portion being disposed at an angle to the grooves of the other surface of the same chamber portion.

8. A device according to any one of claims 5 to 7, further including passageways (250, 252, 254) connecting the chamber portions (218, 228) together to allow them to fill at an approximately equal rate by equalizing any pressure difference between them.

9. A device according to any one of the preceding claims, wherein the liquid is whole blood and the lighter phase is serum or plasma, and further including means (62, 74; 74A; 74C) for withdrawing and trapping residual blood cells left in the lighter phase collecting portion.

10. A device according to claim 2 or 3, wherein the valve means (50; 50A; 50B; 50C; 50D; 50E) comprises a valve seat (58; 58A; 58C; 58D; 58E), a closure member (54; 54A; 90; 54C; 54D; 54E), and biasing means (60; 60A; 91; 60D; 60E) for biasing the closure member (54; 54A; 90; 54C; 54D; 54E) against the valve seat (58; 58A; 58C; 58D; 58E) to close off the first chamber (32; 32A; 32B; 218, 228; 32D; 32E) from the second chamber (64; 64A; 64B; 64C; 64D; 64E), the biasing means (60; 60A; 91; 60D; 60E) being operative in a direction which is substantially perpendicular to the centrifugal force generated during centrifuging, the biasing means (60; 60A; 91; 60D; 60E) having a biasing constant adjusted to allow the closure member (54; 54A; 90; 54C; 54D; 54E) to move off the valve seat (58; 58A; 58C; 58D; 58E) in response only to a predetermined liquid head of pressure.

11. A device according to any one claims 1 to 10, wherein the valve means comprises a ball valve (54; 54A; 54B; 54C).

12. A valve for use in a device according to claim 10, wherein the biasing means comprises a cellular foam having a Young's modulus of no larger than about 345kPa.

13. A valve according to claim 12, wherein the cellular foam comprises an open-celled polyurethane foam having a density of from about 240 to about 320kgm$^{-3}$, a Young's Modulus of from about 40 to about 70kPa, a compression set of less than 2% when measured at 23°C per ASTM 1667, and a Shore "O" durometer test of from about 10 to about 20.

14. A valve according to claim 12 or 13, wherein the closure member (54D; 54E) is selected from an impervious, non-sticking, dimensionally stable material which is sufficiently flexible and thin so as to conform to the valve seat.

15. A valve according to claim 14, wherein the material of the closure member (54D; 54E) comprises a polymer tape no thicker than about 0.2 mm.

16. A valve according to claim 15, wherein the polymer tape is selected from the following polymers each having the following maximum thickness:

| | |
|---|---|
| cellulose acetate | 0.10 mm |
| polyethylene | 0.20 mm |
| polyester | 0.15 mm |
| polyester silvered on one side | 0.15 mm |
| polytetrafluoroethylene | 0.15 mm |
| polycarbonate | 0.15 mm. |

FIG. 1    (PRIOR ART)

FIG. 2B

FIG. 2A

FIG. 3

FIG. 4

EP 0 430 356 A2

*FIG. 5*

*FIG. 6A*

*FIG. 6B*

**FIG.7**

**FIG.8**

**FIG. 9**

**FIG. 10**

FIG. 11

FIG. 12

FIG. 13

FIG. 14

21

*FIG. 15*

*FIG. 16*